# DEMANDE DE BREVET EUROPEEN

(11) **EP 1 308 505 A2**
(43) Date de publication de la demande: **07.05.2003**
(21) Numéro de dépôt: 02292642.2
(22) Date de dépôt: 24.10.2002
(51) Int. Cl.: C12N 1/00, C12N 1/18, C12P 7/06, C12P 7/56, C12P 13/08, C12N 9/00

(54) **Procédé de préparation d'un milieu de fermentation autosuffisant**

(30) Priorité: 30.10.2001 FR 0114090
(71) Demandeur: Roquette Frères, 62136 Lestrem (FR)
(72) Inventeur: Fouache, Catherine, 62113 Sailly Labourse (FR); Segueilha, Laurent, 59130 Lambersart (FR)
(74) Mandataire: Boulinguiez, Didier

(57) **Abrégé**

L'invention est relative à un procédé de préparation d'un milieu de fermentation autosuffisant permettant la production de métabolites à partir d'une matière première renouvelable, caractérisé par le fait qu'il consiste à choisir la matière première renouvelable dans le groupe constitué des solubles de blé, de pois et de pomme de terre, préférentiellement les solubles de blé, traiter ladite matière première renouvelable de manière à en libérer les sources carbonée et azotée directement assimilables par des microorganismes et récupérer le milieu de fermentation autosuffisant ainsi obtenu.

## Description

La présente invention est relative à un procédé particulier de préparation d'un milieu de fermentation autosuffisant à partir d'une matière première renouvelable.

Plus précisément, la présente invention est relative à un procédé particulier de traitement d'une matière première renouvelable, de telle manière qu'il soit possible de l'utiliser directement en fermentation, pour la production de métabolites.

Dans la présente invention, on entend par « matière première renouvelable », les déchets des industries alimentaires peu coûteux, non raffinés, généralement non toxiques et riches en sources d'azote et de carbone. Plus particulièrement, il s'agira des coproduits des amidonneries et plus particulièrement encore des amidonneries de blé, de pois ou de pomme de terre.

On entend également par « milieu de fermentation autosuffisant » un milieu de fermentation renfermant tous les nutriments nécessaires à la croissance des microorganismes et nécessaires et suffisants pour la production de métabolites d'intérêt, sans qu'il ne soit nécessaire de leur ajouter un quelconque complément nutritif.

On entend au sens de l'invention par « métabolites », les produits de transformation par voie fermentaire de sources carbonées directement assimilables par des microorganismes. Il s'agira avantageusement de métabolites choisis dans le groupe constitué par les acides organiques et les acides aminés, et préférentiellement les acides organiques comme l'acide L-Lactique, l'acide gluconique, l'acide citrique, et les acides aminés comme la L-Lysine ou la L-Thréonine.

De manière générale, il est admis que le choix d'une matière première renouvelable comme base destinée à la fermentation pour la production de métabolites d'intérêt est déterminé à la fois par sa disponibilité, son coût et son aptitude à permettre des productivités élevées.

Mais on considère également qu'un milieu de fermentation doit non seulement être constitué par une source carbonée, mais également par une source azotée, auxquelles on ajoute des minéraux et des sels organiques.

Il est admis par les spécialistes du domaine de la fermentation que la « source carbonée » peut-être tirée de matières premières renouvelables comme les mélasses, les hydrolysats d'amidon de blé, de maïs, de riz, de manioc ou des pommes de terre, mais les « sources carbonées directement assimilables » sont les sucres, raffinés ou purifiés à partir desdites sources carbonées, tels que le glucose, le fructose, le maltose, le saccharose, le lactose et les dextrines.

Des exemples de « sources azotées » ou nutriments protéiques sont quant à eux les extraits de levures, la liqueur de trempe du maïs (encore appelée Corn Steep Liquor), le lait non dénaturé, les protéines des mélasses, les extraits de viande ou la farine de soja. Mais il est souvent préféré d'utiliser les extraits de levures comme sources d'azote et également comme compléments de vitamines et d'éléments minéraux.

En toute généralité, le milieu de fermentation constitué d'une « source carbonée directement assimilable » i.e. glucose ou saccharose, et d'extraits de levures, peut être mis en oeuvre basiquement pour bon nombre de fermentations, telles les fermentations conduisant à la production d'acides organiques, tels les acides lactique, propionique, gluconique, citrique..., les acides aminés essentiels tels la lysine ou tout autre métabolite d'intérêt industriel.

Dans la demande de brevet WO 98/54.351, il est ainsi par exemple décrit que pour la préparation du milieu de production de L-Lysine, il peut être choisi une source carbonée sélectionnée dans le groupe constitué du saccharose, mais aussi des mélasses, de l'amidon et des hydrolysats d'amidon issus de diverses sources, comme le maïs et le blé.

Il est cependant obligatoire d'y ajouter une source d'azote, choisie dans le groupe constitué des extraits de levure ou encore des mélasses, des protéines, des peptides et des aminoacides, du corn steep ou des solubles de blé.

Dans le domaine des acides organiques, il est par exemple choisi, pour la production de l'acide lactique :
- dans le brevet US 5.416.020, un procédé de production d'acide L-Lactique à partir de perméat de petit lait et de petit lait, auquel il est encore ajouté de l'extrait de levures en présence de manganèse divalent, avec un mutant de *Lactobacillus delbrueck*ii sub. *bulgaricus* ATCC 55163 qui produit essentiellement de l'acide L-Lactique.

Le perméat de petit lait contient bien de 75 à 80 % en poids de lactose, mais ne renferme plus de protéines de grande taille. Il est donc déficient en source d'azote essentielle pour la croissance des microorganismes, d'où le complément rendu nécessaire en extraits de levures. Le petit lait ajouté renferme essentiellement de l'ordre de 65 à 75 % en poids de lactose.

L'extrait de levures fournit alors au milieu de fermentation les nutriments qui ne sont pas apportés de manière adéquate par les perméats de petit lait et le petit lait lui-même.
- Dans le brevet US 4.467.034, il est montré qu'il est possible de produire de l'acide lactique à partir du petit lait comme matière première, grâce à un nouveau *Lactobacillus bulgaricus* DSM 2129. Le petit lait doit cependant encore être complémenté par une source d'azote, i.e. de l'extrait de viande, du corn steep ou de la farine de soja, et également par des vitamines et des sels minéraux.

Dans ces conditions, la mise en oeuvre de ces milieux de fermentation nécessite des combinaisons complexes respectant le rapport azote / carbone, plus l'ajout de compléments nécessaires à une productivité efficace des microorganismes d'intérêt.

Ces milieux sont également mis en oeuvre pour la production de biomasse (par exemple pour la préparation de ferments lactiques), mais présentent également le même ordre de difficultés, tenant aux exigences métaboliques des microorganismes considérés.

Par ailleurs, il est admis que ces milieux présentent en plus l'inconvénient de ne pas être extrapolables à la production à l'échelle industrielle de ces mêmes métabolites d'intérêts (difficulté de disposer de milieux standardisés quant à leur composition et coûts supplémentaires amenés par les étapes de purification ultérieures).

De tout ce qui précède, il résulte qu'il existe un besoin non satisfait de disposer d'un procédé simple et efficace permettant de préparer un milieu de fermentation sans mettre en oeuvre des étapes lourdes, nombreuses et coûteuses, tant notamment au niveau du choix des sources carbonée et azotée du milieu de fermentation que de leur association pour fabriquer un milieu de fermentation équilibré, adapté à la fermentation considérée.

Soucieuse de développer un procédé qui permette de répondre mieux que ceux qui existent déjà aux contraintes de la pratique, la société Demanderesse a constaté que cet objectif pouvait être atteint par un procédé consistant à préparer un milieu de fermentation autosuffisant directement à partir d'une matière première renouvelable.

Plus particulièrement, le procédé de préparation d'un milieu de fermentation permettant la production de métabolites à partir d'une matière première renouvelable conforme à l'invention de la société Demanderesse est caractérisé par le fait qu'il consiste à :
- choisir la matière première renouvelable dans le groupe constitué des solubles de blé, de pois et de pomme de terre, préférentiellement les solubles de blé,
- traiter ladite matière première renouvelable de manière à en libérer les sources carbonée et azotée directement assimilables par des microorganismes,
- récupérer le milieu de fermentation autosuffisant ainsi obtenu.

La première étape du procédé conforme à l'invention consiste donc à choisir la matière première renouvelable dans le groupe constitué des solubles de blé, de pois et de pomme de terre, préférentiellement les solubles de blé.

La société Demanderesse a ainsi vaincu le préjugé technique selon lequel la préparation d'un milieu de fermentation doit obligatoirement s'entendre de la reconstitution d'un milieu de fermentation composite, principalement à partir d'une source carbonée et d'une source azotée d'origines séparées, avec l'ajout de vitamines, de facteur de croissance et d'oligo-éléments.

Comme il a été dit plus haut, il est en effet considéré classiquement dans l'état de la technique qu'il faut disposer dudit milieu de fermentation composite pour obtenir la production d'un métabolite d'intérêt avec de bons rendement et productivité.

Cependant, cette nécessité s'accompagne surtout d'un inconvénient majeur, qui réside dans son coût élevé, et d'une difficulté, qui est de l'adapter aux exigences des microorganismes producteurs.

Après de nombreuses recherches longues et fastidieuses, il est du mérite de la société Demanderesse d'avoir trouvé dans les matières premières renouvelables, et plus particulièrement dans les résidus des amidonneries de blé, de pois et de pomme de terre, le milieu de fermentation adapté aux exigences de la pratique.

Plus particulièrement, et là encore en allant à l'encontre d'un autre préjugé technique, la société Demanderesse a trouvé que cette matière première renouvelable devait être avantageusement choisie dans le groupe constitué des solubles de blé, de pois ou de pomme de terre, alors qu'il est communément admis que ces solubles ne constituent en tant que tels qu'une source d'azote pour la constitution des milieux de fermentation.

Il a ainsi été trouvé que ces solubles pouvaient être directement utilisés, après le traitement conforme à l'invention par la société Demanderesse, non seulement comme une source d'azote, mais également comme une source unique de carbone et d'oligo-éléments, voire de vitamines, nécessaire et suffisante pour la fermentation de tout microorganisme d'intérêt.

Les solubles de blé, par exemple, proviennent du flux de séparation des amidons de blé « B » résultant de la séparation de l'amidon dans le procédé de l'amidonnerie de blé humide.

L'amidon B ou amidon second est l'amidon constitué essentiellement par une proportion prépondérante de petits granules d'amidon ou de granules endommagés.

A côté de cet amidon B, il est connu que les solubles de blé renferment également des quantités non négligeables de protéines de haut poids susceptibles de constituer une source d'azote pour des microorganismes d'intérêt.

Quant aux solubles de pomme de terre, il sont obtenus par récupération de la fraction soluble issue de l'écrasage des pommes de terre en début d'extraction de la fécule.

Les solubles de pois résultent de l'eau de trempage du pois et sont récupérés avant l'écrasage et la séparation des différents constituants du pois.

La seconde étape du procédé conforme à l'invention consiste donc à traiter ladite matière première renouvelable de manière à en libérer les sources carbonée et azotée directement assimilables par des microorganismes.

Les matières premières renouvelables contiennent ici à la fois de l'amidon comme source de carbone ou du glucose, et des protéines de haut poids moléculaire, à côté de peptides et acides aminés libres comme source d'azote.

Cependant, s'il est admis que certains microorganismes ont la capacité d'assimiler directement l'amidon ou les protéines de haut poids moléculaire, car ils disposent de l'équipement enzymatique nécessaire à leur dégradation pour leur croissance et pour la production de métabolites d'intérêt, pour d'autres microorganismes, il est nécessaire de les placer dans des conditions où les sources carbonées et azotées sont traitées de manière à être directement assimilables.

Ces traitements sont donc à adapter en fonction de la physiologie des microorganismes d'intérêt.

Dans un premier mode de réalisation du procédé conforme à l'invention, pour des microorganismes par exemple dépourvu d'amylases, il est avantageusement procédé à la libération des sucres fermentescibles des solubles par chauffage desdits solubles à une température d'au moins 60°C, par traitement au moyen d'une α-amylase et d'une glucoamylase et éventuellement au moyen d'une enzyme susceptible de dégrader les polysaccharides pariétaux d'origine végétale choisie dans le groupe des hémicellulases, pectinases et xylanases.

Dans un deuxième mode de réalisation du procédé conforme à l'invention, pour des microorganismes incapables d'assimiler les protéines de haut poids moléculaire, il est avantageusement procédé à la libération des acides aminés et/ou des peptides assimilables des solubles par traitement avec des enzymes protéolytiques choisies par exemple dans le groupe constitué des protéases alcalines et des protéases acides.

Un traitement à pH 7 et à une température de 60°C pendant environ 6 h, à une dose de 1 % /sec peut être avantageusement mis en oeuvre pour le traitement avec les protéases alcalines.

Quant aux protéases acides qui conviennent pour réaliser la protéolyse, elles sont à choisir dans le groupe des pancréatine, trypsine, chymotrypsine...

Un traitement à pH 4,5 et à une température de 60°C pendant environ 6 h, à une dose de 1 %/sec peut être avantageusement mis en oeuvre pour le traitement avec les protéases acides.

Cette étape de protéolyse forme des peptides qui peuvent en outre avoir un effet activateur vis-à-vis de certains microorganismes.

Enfin, dans un troisième mode de réalisation du procédé conforme à l'invention, ces deux traitements des solubles par liquéfaction et saccharification d'une part, et par protéolyse d'autre part, peuvent être mis en oeuvre pour la fermentation de microorganismes incapables d'assimiler directement les sources carbonées et azotées telles que présentes dans lesdits solubles.

Comme il sera exemplifié ci-après, le contenu en oligo-éléments, voire en vitamines, des solubles les rendent particulièrement attractifs pour bon nombre de fermentations.

La troisième étape du procédé conforme à l'invention consiste donc à récupérer le milieu de fermentation autosuffisant ainsi obtenu et à l'utiliser directement en fermentation.

Il peut être avantageusement choisi de soumettre le milieu de fermentation autosuffisant à une étape supplémentaire de microfiltration pour en éliminer les impuretés insolubles.

Cette étape peut être effectuée en utilisant tout moyen connu par ailleurs de l'homme du métier, telle la microfiltration sur membranes dont la taille des pores est adaptée à la taille desdites impuretés insolubles. Il peut ainsi être par exemple utilisé une membrane de 0,14 µm.

Ces milieux sont particulièrement adaptés pour la production d'acide lactique, de lysine, d'éthanol, d'enzymes, pour la production de polysaccharides choisis dans le groupe des pullulans et des dextrans et également pour la production de populations de microorganismes y relatives comme par exemple les ferments lactiques ou les levures.

Il est enfin également du mérite de la société Demanderesse de proposer une solution de mise en oeuvre particulière du milieu de fermentation autosuffisant conforme à l'invention s'il est souhaité de récupérer les métabolites produits sans qu'il ne soit nécessaire de mettre en oeuvre des étapes lourdes et coûteuses de purification.

En effet, il est communément admis par exemple dans le cas de l'utilisation des solubles de blé, que l'amidon B ou amidon second contient des impuretés telles que les pentosanes et des lipides.

Ces impuretés, dont certaines échappent aux traitements classiques de purification et de déminéralisation, se retrouvent dans les hydrolysats de ces amidons, et rendent ainsi l'amidon B impropre, par exemple, à la fabrication du dextrose de qualité alimentaire. C'est la raison pour laquelle il est considéré qu'il ne peut être que difficilement trouvé des débouchés industriels pour de tels amidons « B ».

Dans ces conditions, il est du mérite de la société Demanderesse, non seulement d'avoir mis au point un procédé permettant de traiter ces solubles de blé de manière à produire un milieu de fermentation autosuffisant, mais également de proposer une solution à la préparation de métabolites d'une qualité telle qu'il ne sera pas nécessaire de leur appliquer des méthodes trop astreignantes de purification.

Cette solution consiste à abaisser en contenu le milieu de fermentation autosuffisant et de le complémenter en une source de carbone directement assimilable, de manière à apporter au microorganisme considéré la quantité de carbone qui lui est nécessaire à la fois pour sa croissance et pour permettre la production de métabolites d'intérêt, sans perte de rendement ou de productivité.

La part résiduelle du milieu de fermentation autosuffisant doit être cependant réglée pour maintenir les apports en azote, sels minéraux et vitamines indispensables aux microorganismes, comme il sera exemplifié ci-après pour la fermentation lactique.

D'autres caractéristiques et avantages de l'invention apparaîtront à la lecture des exemples qui suivent. Ils ne sont toutefois donnés ici qu'à titre illustratif et non limitatif.

### Exemple 1

Des solubles de blé à 20 % de matière sèche, provenant du flux de la séparation des amidons de blé « B » sont chauffés à 60°C pendant 12 h et traités au moyen d'une α-amylase TERMAMYL LC de NOVO à raison de 0,05 %/sec.

A partir des solubles de blé ainsi traités, trois milieux de fermentation autosuffisants peuvent être obtenus, le premier résultant de la saccharification de l'amidon des solubles de blé ainsi liquéfiés (produit A), le deuxième résultant du traitement des solubles de blé liquéfiés avec des protéases (produit B), le troisième résultant des deux traitements ci-avant mentionnés (produit C).

Pour la préparation du produit A, les solubles ainsi liquéfiés sont amenés à une MS comprise entre 15 et 20 % et traités pendant 3 à 5 heures à 60°C au moyen d'une amyloglucosidase OPTIDEX L 300 A de GENENCOR à raison de 0,5 %/sec et d'une hémicellulase SPEZYME CP de GENENCOR à raison de 0,3 %/sec pour libérer les sucres fermentescibles. Les insolubles sont éliminés par microfiltration sur membrane de 0,14 µm.

Le produit A d'une matière sèche de 14,6 % conforme à l'invention présente la composition figurée dans le tableau I suivant.

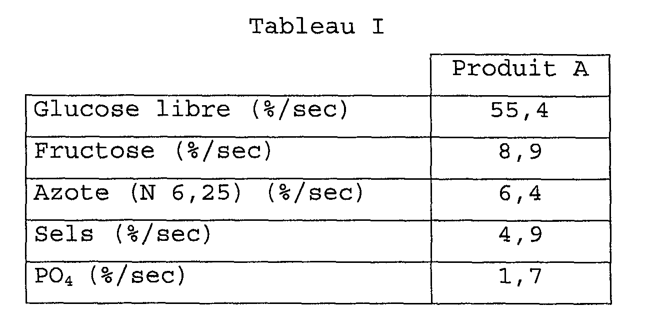

Pour préparer le produit B, les solubles de blé liquéfiés sont amenés également à une MS comprise entre 15 et 20 % (le pH est ajusté à une valeur comprise entre 7,5 et 8 par de la soude 1 fois normale), et traités à 60°C pendant 4 à 6 heures au moyen de l'ALCALASE Novo à raison de 0,2 à 1 %/sec. Le produit obtenu présente un pH final de l'ordre de 6,5 à 7. Les insolubles sont éliminés par microfiltration sur membrane de 0,14 µm.

Le produit B d'une matière sèche de 14,8 % conforme à l'invention présente la composition figurée dans le tableau II suivant.

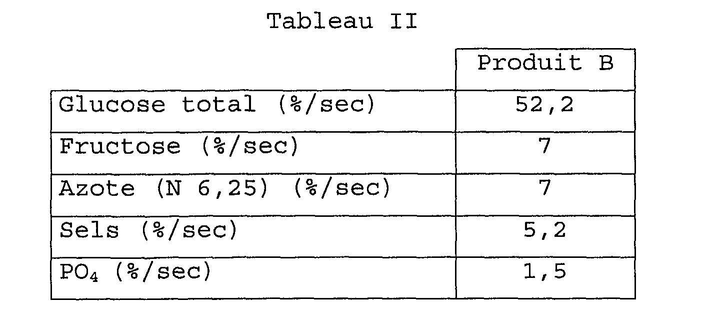

Pour préparer le produit C, les solubles de blé subissent d'abord une saccharification dans les mêmes conditions que pour la préparation du produit A, puis un traitement au moyen de l'ALCALASE dans les mêmes conditions qui permettent d'obtenir le produit B. Les insolubles sont également éliminés par microfiltration sur membrane de 0,14 µm.

Le produit C d'une matière sèche de 18,4 % conforme à l'invention présente la composition figurée dans le tableau III suivant.

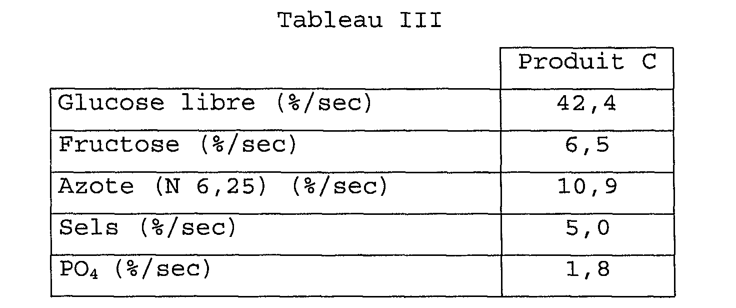

Les aminogrammes réalisés sur ces trois milieux de fermentation autosuffisants conformes à l'invention base solubles de blé montrent un contenu remarquable en acides aminés acides et acides aminés à radicaux non polaires, i.e. respectivement de l'ordre de 2250 et 1050 mg/kg de MS.

### Exemple 2

A partir des solubles de pomme de terre, en utilisant le mode de traitement décrit dans l'exemple 1 pour le produit A, on obtient le milieu de fermentation autosuffisant D suivant.

Le tableau IV suivant présente les profils de ce milieu de fermentation autosuffisant.

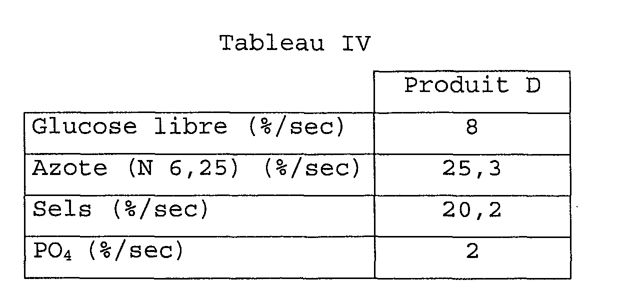

Les aminogrammes réalisés sur ce milieu de fermentation autosuffisant conforme à l'invention montre un contenu remarquable en acides aminés acides et acides aminés à radicaux non polaires, i.e. respectivement de l'ordre de 2730 et 1100 mg/kg de MS.

En outre, les milieux de fermentation ainsi obtenus sont riches en vitamine B7 (teneur de 4 fois supérieure à ce que renferment classiquement les extraits de levures) et en vitamine B3.

### Exemple 3

A partir des solubles de pois, en utilisant le mode de traitement décrit dans l'exemple 1 pour le produit A, on obtient le milieu de fermentation autosuffisant E suivant.

Le tableau V suivant présente les profils de ce milieu de fermentation autosuffisant.

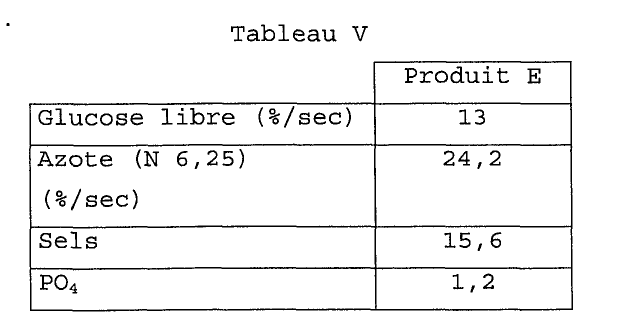

Les aminogrammes réalisés sur le milieu de fermentation autosuffisant conforme à l'invention montrent un contenu remarquable en acides aminés à radicaux basiques et acides aminés à radicaux acides, i.e. respectivement de l'ordre de 13150 et 26780 mg/kg de MS.

### Exemple 4

Le tableau VI suivant présente les rendement et productivité en acide L-lactique obtenus en fermenteur de 15 1 de volume utile avec 13 1 de solubles de blé traités en suivant le procédé conforme à l'invention (Produits A à C de l'exemple 1).

En témoin « milieu standard », un milieu de fermentation composé de glucose à 80 g/l, d'extraits de levures à 10 g/l et de (NH₄)₂HPO₄ à 0,5 g/l est testé.

La matière sèche de 150 à 180 g/l des milieux de fermentation autosuffisants conformes à l'invention utilisés est choisie de manière à ce que lesdits milieu renferment de l'ordre de 80 g/l d' « équivalent glucose ».

En fonction du mode de traitement des solubles de blé, ces « équivalents carbone ou azote » seront donc directement assimilables ou non pour le microorganisme considéré.

Un milieu de fermentation autosuffisant équivalent au produit C de l'exemple 1 mais non microfiltré (appelé produit C « brut ») est également testé en témoin « non microfiltré ».

1,5 l de milieu d'une préculture de 7 h d'une souche de *Lactococcus lactis* sont utilisés pour inoculer ces fermenteurs.

Le pH, fixé à 6,5, est régulé avec NH₄OH 12N. La température est de 40°C.

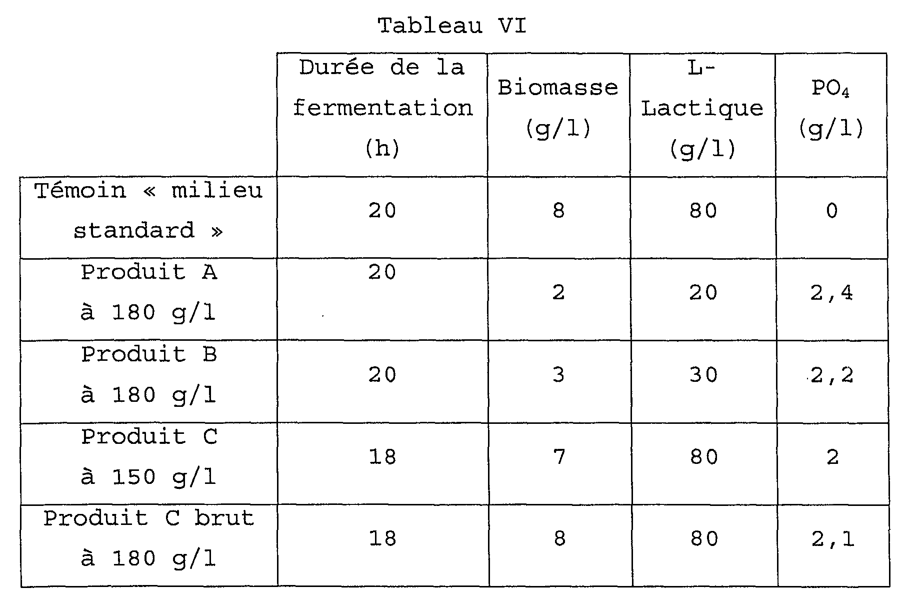

Ce tableau montre que pour un microorganisme du genre *Lactococcus lactis* producteur d'acide L-lactique, un milieu autosuffisant à base de solubles de blé uniquement, où les sources carbonée et azotée sont rendues directement assimilables par un traitement adéquat (en l'occurrence liquéfaction et saccharification de l'amidon « B » de blé et protéolyse à l'aide de l'ALCALASE du contenu en protéines) permet d'obtenir un rendement et une productivité au moins équivalents avec ce qui est obtenu en utilisant un milieu de production standard beaucoup plus coûteux à base de glucose pur et d'extraits de levures.

Les solubles de blé liquéfiés, saccharifiés et protéolysés peuvent donc être avantageusement mis en oeuvre pour la fermentation lactique. L'essai réalisé avec le produit C non microfiltré montre également que pour cette fermentation particulière, les impuretés insolubles ne gênent en rien le rendement ni la productivité en acide L-lactique.

Enfin, la détermination de la biomasse a été réalisée en fin de fermentation pour tous les essais entrepris.

Il apparaît clairement que les solubles traités selon le procédé conforme à l'invention, en l'occurrence par liquéfaction, saccharification et protéolyse se révèlent aussi efficaces pour la croissance de *L. lactis* que le milieu standard reconstitué. Le milieu autosuffisant conforme à l'invention est donc bien adapté à la production de biomasse, en particulier ici pour la production de ferments lactiques.

### Exemple 5

Il peut être déploré d'utiliser trop de solubles de blé en regard de la qualité du métabolite produit, et par là même de la possibilité de développer une technologie lourde de purification.

Il est possible de limiter l'apport en solubles de blé traités en suivant le procédé conforme à l'invention, par la maîtrise des conditions de fermentation, i.e. par la parfaite connaissance des besoins nutritionnels du ou des microorganismes en question.

Dans le cas de la production d'acide L-lactique avec *Lactococcus lactis*, tel que présentée dans l'exemple 4, il est possible de ramener la matière sèche du produit C à 40 g/l (« équivalent glucose de 20 g/l ») et de compenser la baisse en source carbonée nécessaire par l'ajout de 85 g/l de glucose (pouvant par exemple être produite à partir d'hydrolysat d'amidon de blé).

Le tableau VII suivant présente les résultats obtenus.

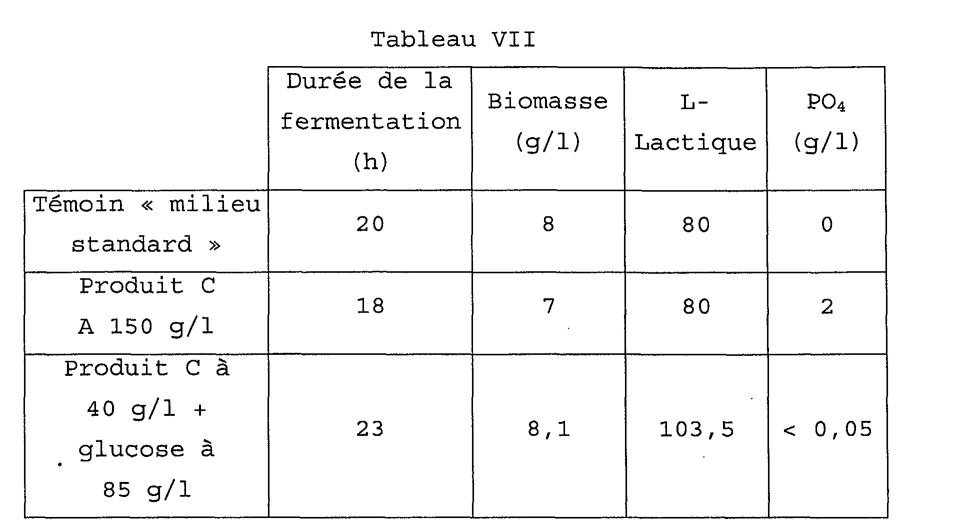

L'apport « résiduel » en azote, en source carbonée et en oligo-éléments permet de conserver des rendements et productivité équivalents pour une charge en impuretés diminuée d'un facteur 3,75.

### Exemple 6

La fermentation des levures de type *S. cerevisiae* est classiquement mise en oeuvre pour la production d'éthanol.

Il est étudié la production de biomasse de levures en utilisant le milieu de fermentation autosuffisant conforme à l'invention en comparaison avec un milieu classique constitué d'extraits de levures comme source d'azote, de glucose comme source carbonée, complémenté par des sels.

La production de biomasse de *S. cerevisiae* est réalisée dans un milieu préparé à partir du produit A de l'exemple 1 à raison de 80 g/l.

Le milieu témoin est constitué de Glucose à 45 g/l, d'extraits de levure à 5 g/l, de (NH₄)₂SO₄ à 10 g/l, de KH₂PO₄ à 5 g/l et de MgSO₄ à 2 g/l.

Le pH est régulé à 5 avec de la soude normale, la température est fixée à 30°C et la culture est réalisée en réacteur de 2 l sous une agitation de 600 rpm et une aération de 1 vvm.

Le tableau VIII suivant présente le résultat de croissance des levures au cours du temps pour les deux milieux de fermentation.

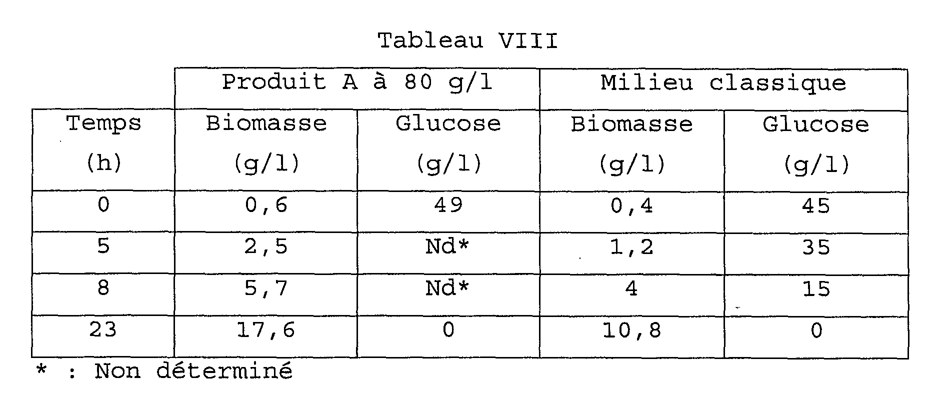

Ce résultat montre par là même que le milieu de fermentation autosuffisant conforme à l'invention est particulièrement bien adapté à la production de biomasse de levure, et par extension, à toutes productions de métabolites dont la synthèse est concomitante à la croissance desdites levures, comme dans le cas de la production d'éthanol.

### Exemple 7

Il est étudié la production d'éthanol en utilisant le milieu de fermentation autosuffisant conforme à l'invention en comparaison avec un milieu classique constitué d'extraits de levures comme source d'azote, de glucose comme source carbonée, complémenté par des sels.

La production de biomasse de *S. cerevisiae* est réalisée dans un milieu préparé à partir du produit A de l'exemple 1 à raison de 180 g/l.

Le milieu témoin est constitué de Glucose à 10 g/l, d'extraits de levure à 5 g/l, de (NH₄)₂SO₄ à 10 g/l, de KH₂PO₄ à 5 g/l et de MgSO₄ à 2 g/l.

Le pH est régulé à 5 avec de la soude normale, la température est fixée à 30°C et la culture est réalisée en réacteur de 15 l sous une agitation de 200 rpm.

Le tableau IX suivant présente le résultat de croissance des levures au cours du temps pour les deux milieux de fermentation.

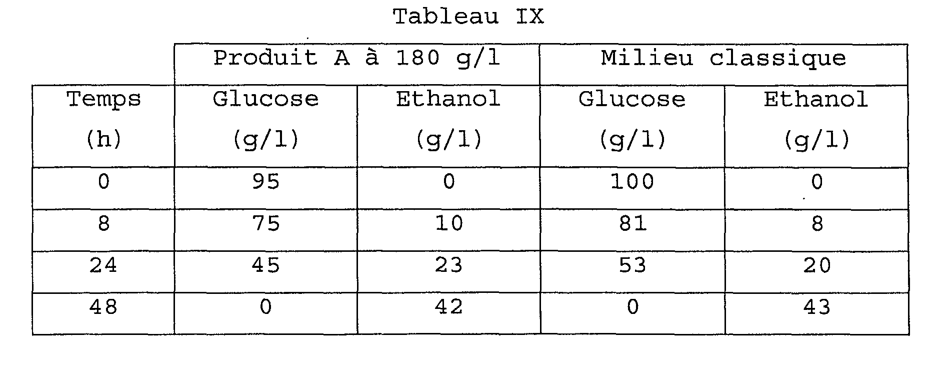

Le milieu de fermentation autosuffisant conforme à l'invention est donc particulièrement bien adapté à la production d'éthanol.

## Revendications

1. Procédé de préparation d'un milieu de fermentation autosuffisant permettant la production de métabolites à partir d'une matière première renouvelable, **caractérisé par le fait qu'**il consiste à :
- choisir la matière première renouvelable dans le groupe constitué des solubles de blé, de pois et de pomme de terre, préférentiellement les solubles de blé,
- traiter ladite matière première renouvelable de manière à en libérer les sources carbonée et azotée directement assimilables par des microorganismes,
- récupérer le milieu de fermentation autosuffisant ainsi obtenu.

2. Procédé selon la revendication 1, **caractérisé par le fait que** l'on soumet le milieu de fermentation autosuffisant à une étape de microfiltration pour en éliminer les impuretés insolubles.

3. Procédé selon l'une ou l'autre des revendications 1 et 2, **caractérisé par le fait que** l'on traite la matière première renouvelable par des enzymes de liquéfaction et de saccharification de l'amidon de manière à en libérer le contenu en source carbonée directement assimilable.

4. Procédé selon l'une quelconque des revendications 1 à 3, **caractérisé par le fait que** l'on traite la matière première renouvelable par des enzymes protéolytiques choisies dans le groupe constitué des protéases alcalines de manière à en libérer le contenu en source azotée directement assimilable.

5. Procédé de préparation d'un milieu de fermentation permettant la production de métabolites aisément purifiables à partir d'un milieu de fermentation autosuffisant préparé ' en suivant le procédé d'une quelconque des revendications 1 à 4, **caractérisé en ce qu'**il est complémenté en une source de carbone directement assimilable.

6. Milieu de fermentation susceptible d'être obtenu selon l'une quelconque des revendications 1 à 5.

7. Utilisation du milieu de fermentation selon la revendication 6 pour la production d'acide lactique.

8. Utilisation du milieu de fermentation selon la revendication 6 pour la production de lysine.

9. Utilisation du milieu de fermentation selon la revendication 6 pour la production de polysaccharides choisis dans le groupe des pullulans et des dextrans, et plus préférentiellement des pullulans.

10. Utilisation du milieu de fermentation selon la revendication 6 pour la production de populations de microorganismes.

11. Utilisation du milieu de fermentation selon la revendication 6 pour la production d'enzymes.

12. Utilisation du milieu de fermentation selon la revendication 6 pour la production d'éthanol.
